# EUROPÄISCHE PATENTSCHRIFT

(11) **EP 0 423 534 B1**
(45) Veröffentlichungstag und Bekanntmachung des Hinweises auf die Patenterteilung: **14.09.1994**
(21) Anmeldenummer: 90118834.2
(22) Anmeldetag: 02.10.1990
(51) Int. Cl.: A61B 17/22

(54) **Lithotriptor mit Ortungseinrichtung**
Lithotripter with locating device
Lithotripteur avec dispositif de localisation

(30) Priorität: 12.10.1989 DE 3934105
(43) Veröffentlichungstag der Anmeldung: 24.04.1991
(73) Patentinhaber: Richard Wolf GmbH, 75438 Knittlingen (DE)
(72) Erfinder: Belikan, Thomas, W-7134 Knittlingen (DE); Krauss, Werner, W-7134 Knittlingen (DE); Wurster, Helmut, W-7519 Oberderdingen (DE)
(74) Vertreter: Wilcken, Thomas, Dipl.-Ing.

(56) Entgegenhaltungen:
- EP-A- 0 227 929
- EP-A- 0 372 174
- DE-A- 3 503 702

## Beschreibung

Die Erfindung geht aus von einem Lithotriptor gemäß dem Oberbegriff des Patentanspruches 1, wie er aus der Druckschrift EP-A-0 227 929 bekannt ist.

In der EP-A-0 227 929 ist ein Lithotriptor der vorgenannten Art beschrieben. Bei diesem Lithotriptor endet der Ausgang des Röntgenstrahlers vor der das flüssige Ankopplungsmedium abschließenden Membran. Um den Ausgang des Röntgenstrahlers herum sind mehrere, mit einem Fluid füllbare und evakuierbare Bälge vorgesehen, die sich zwecks einer sicheren Röntgenortung des Konkrementes zur exakten Positionierung des Patienten in Strahlrichtung ausdehnen und nach Positionierung des Patienten in den Fokus des Ultraschallwandlers in entgegengesetzter Richtung kollabieren. Der Röntgenstrahl durchdringt somit zumindest teilweise die mit dem Fluid gefüllten Bälge und wird dadurch geringer gedämpft als bei ausschließlicher Durchdringung des Ankopplungsmediums. Des weiteren steht die durch ein Wasserkissen gebildete Vorlaufstrecke dieses Lithotriptors über eine Verbindungsleitung mit einem zweiten, im wesentlichen identischen Wasserkissen in Verbindung, das wiederum auf einem Hubtisch montiert ist und von einer vorbestimmten Kraft einer Feder des Hubtisches beaufschlagt wird. Dadurch wird der Druck im ersten Wasserkissen bei der Behandlung des Patienten mit Stoßwellen konstant gehalten.

In der EP-A-0 372 174 ist ebenfalls ein Lithotriptor der einleitend angeführten Art beschrieben, der jedoch einen Stand der Technik nach Art. 54 (3) EPÜ darstellt. Bei diesem Lithotriptor ist an den Ausgang des Röntgenstrahlers ein einzelner, aufblasbarer Ballon angeschlossen, um den Weg für die Röntgenstrahlen des Röntgenstrahlers vom Kopplungsmedium der Vorlaufstrecke des Ultraschallwandlers freizumachen. Das Gas zum Aufblasen des Ballons kann einerseits direkt in den Ballon, andererseits aber auch über einen vorderen Abschnitt des Röntgenstrahlers indirekt in den Ballon eingeleitet werden.

Die Aufgabe der Erfindung besteht darin, die Vorrichtung, die eine Dämpfung der zur Ortung dienenden Röntgenstrahlen bei Lithotriptoren der eingangs erwähnten Art ganz oder nahezu ausschließen, derart zu gestalten, daß das während der Evakuierung vom gefüllten Ballon eingenommene Volumen wieder mit Ankopplungsmedium automatisch gefüllt wird.

Diese Aufgabe wird durch die Kennzeichenmerkmale des Patentanspruches 1 gelöst.

Durch diese Lösung wird der Ballon mit Luft bzw. Gas so weit aufgeblasen, daß er sich bis oder nahezu bis an die Abschlußmembran der flüssigen Vorlaufstrecke aufbläht, so daß zwischen dem Ausgang des Röntgenstrahlers und der abschließenden Membran sich keine oder nur eine geringe Menge des flüssigen Ankopplungsmediums befindet, d. h. der Röntgenstrahl druchläuft nur noch das Füllgas oder die Luft des Ballons, was keine oder nur eine vernachlässigbare Dämpfung auf die Strahlung ergibt. Der Ballon wird nach der Ortung eines Konkrementes evakuiert, sobald der Stoßwellenwandler eingeschaltet wird, um dessen Stoßwellen nicht zu stören.

Um während der Evakuierung des Gases oder der Luft aus dem Ballon das vom gefüllten Ballon eingenommene Volumen der Vorlaufstrecke wieder mit Ankopplungsmedium automatisch zu füllen, geht man des weiteren so vor, daß für das Aufblasen und Evakuieren des Ballons ein durch eine nachgiebige Teilung in zwei Kammern unterteilter Ausgleichsbehälter vorgesehen ist, dessen eine Kammer an das Ankopplungsmedium der Vorlaufstrecke angeschlossen ist, während dessen andere Kammer mit dem Ballon zu dessen wahlweiser Evakuierung oder Füllung mit Gas bzw. Luft verbunden ist. Dabei geht man vorzugsweise so vor, wie es in dem Anspruch 4 dargelegt ist.

Hierdurch wird das Volumen des Ballons beim Evakuieren automatisch durch die Ankopplungsflüssigkeit aus dem Auslgeichsbehälter ersetzt, und umgekehrt wird beim Orten diese Ankopplungsflüssigkeit durch das Aufpumpen des Ballons mit Gas oder Luft aus dem Ausgleichsbehälter ersetzt.

Weitere vorteilhafte Ausgestaltungen dieser Lösung sind in den Unteransprüchen unter Schutz gestellt.

Die Erfindung ist nachstehend anhand eines Ausführungsbeispieles in der anliegenden Zeichnung erläutert, in der das Schema eines Lithotriptors mit den Merkmalen der Erfindung schematisch und teilweise in Perspektive dargestellt ist.

Der Lithotriptor besteht in bekannter Weise aus einem Ultraschallwandler 1 oder Stoßwellenwandler, dessen fokussierende Kalotte mit einer Vorlaufstrecke aus einem flüssigen Ankopplungsmedium 2 versehen ist, welches durch eine Membran 3 abgeschlossen ist. In der Wandung der Kalotte ist eine Ortungseinrichtung aus einem Röntgenstrahler 4 bzw. es sind darin gegebenenfalls zusätzlich eine oder zwei Ultraschallortungseinrichtungen 5 gelagert.

An den Ausgang des Röntgenstrahlers 4 oder an einen sich an den Ausgang des Strahlers 4 anschließenden, mit Gas gefüllten Tubus 6 ist ein Ballon 7 abgedichtet angeschlossen, der so ausgebildet ist, daß er sich beim Aufblasen mit Luft bzw. Gas in Richtung des Röntgenstrahlers aufweitet und bis oder nahezu bis an die Abschlußmembran 3 der Vorlaufstrecke 2 erstreckt. Dadurch tritt der ortende Röntgenstrahl, lediglich durch Gas verlaufend durch die durchsichtige Membran 3 direkt ohne jegliche Dämpfung in die Körperhöhle ein.

Das Aufblasen des Ballons 7 erfolgt zum Beispiel durch eine Luft- bzw. Gaspumpe, durch die auch die Evakuierung vorgenommen werden kann.

Für das Aufblasen und Evakuieren des Ballons 7 ist ein Ausgleichsbehälter 8 vorgesehen, der durch eine nachgiebige Trennwand 9 in zwei Kammern 10 und 11 unterteilt ist. Die Kammer 10 steht über eine Leitung 12 mit der Ankoppelflüssigkeit 2 der Vorlaufstrecke in Verbindung und kann aus einer Leitung 13 über ein Ventil 14 mit Flüssigkeit ergänzt werden.

Die Gaskammer 11 steht über die Leitung 15, das Schaltventil 16 und die zum Ballon 7 fördernde Pumpe 17 mit dem Ballon 7 in Verbindung, um den Ballon mit Gas bzw. Luft zu füllen. Nach Abschalten der Pumpe 7 und Sperren des Ventils 16 kann der Ballon 7 zur anschließenden Zertrümmerung von georteten Konkrementen über die Leitung 18 durch die Pumpe 19 und das Ventil 20 evakuiert werden, wobei das Gas aus dem Ballon 7 in die Kammer 11 gefördert wird, die sich aufweitet und Flüssigkeit aus der Kammer 10 in die Vorlaufstrecke drückt, womit sich das durch Evakuierung des Ballons in der Vorlaufstrecke entstehende Vakuum mit Ankopplungsflüssigkeit 2 füllt. Umgekehrt wird diese Flüssigkeit beim Füllen des Ballons 7 mit Gas in die Kammer 10 zurückgedrückt.

Der Druck in der Kammer 11 und damit in der Vorlaufstrecke wird über einen Druckregler 21 überwacht, durch den der Sollwert des Gasdruckes gesteuert veränderbar ist, um die Füllmenge der Kammer 11 und somit die Höhe der Membran 3 entsprechend der Tiefe des zu behandelnden Steines im Patienten dem Fokusabstand 22 anzupassen und die Kopplung der Membran 3 mit der Haut des Patienten zu erzielen.

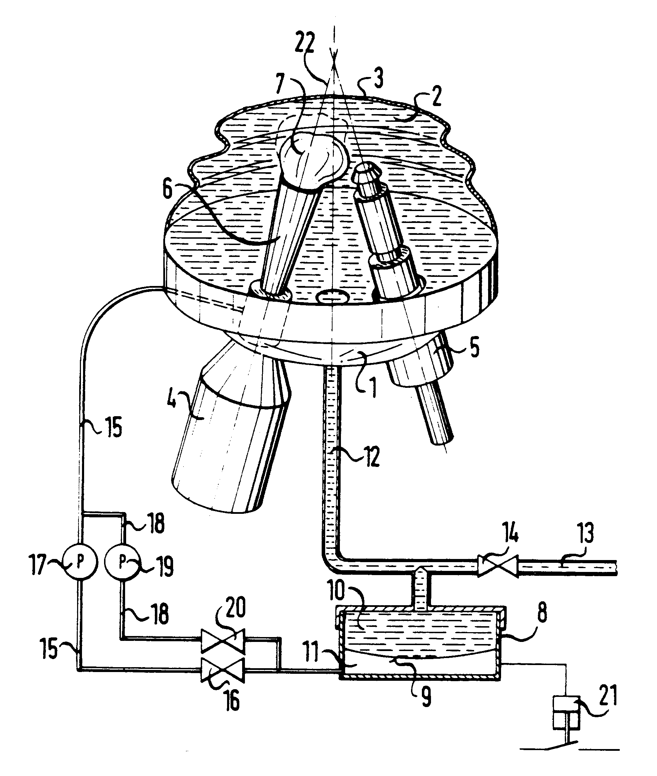

## Patentansprüche

1. Lithotriptor mit einer an einen Patienten anlegbaren, durch eine Membran (3) abgeschlossenen und mit einem flüssigen Ankopplungsmedium (2) gefüllten Vorlaufstrecke eines Stoßwellenwandlers (1) und mit einer für das durch den Stoßwellenwandler zu zerstörende Konkrement oder Gewebe dienenden Ortungseinrichtung aus einem durch die Vorlaufstrecke gerichteten Röntgenstrahler (4), wobei um den Ausgang des Röntgenstrahlers (4) in der Vorlaufstrecke ein röntgentransparenter, mit Gas füllbarer und evakuierbarer Ballon (7) abgedichtet festgelegt ist, der in Strahlrichtung der Ortungseinrichtung in der Vorlaufstrecke (2) entfaltbar ist, dadurch gekennzeichnet, daß für das Füllen und Evakuieren des Ballons (7) mit bzw. von Gas ein durch eine nachgiebige Trennwand (9) in zwei Kammern (10, 11) unterteilter Ausgleichsbehälter (8) vorgesehen ist, dessen eine Kammer (10) an das Ankopplungsmedium (2) der Vorlaufstrecke angeschlossen ist, während die andere Kammer (11) mit dem Ballon (7) zu dessen wahlweiser Evakuierung oder Füllung mit Gas verbunden ist.

2. Lithotriptor nach Anspruch 1, dadurch gekennzeichnet, daß der Ballon (7) an das offene Ende eines sich an den Ausgang des Röntgenstrahlers (4) anschließenden, mit dem Gas füllbaren Tubus (6) angeschlossen ist.

3. Lithotriptor nach Anspruch 1 oder 2, dadurch gekennzeichnet, daß die Gaskammer (11) des Ausgleichsbehälters (8) zur Gaszuführung zum Ballon (7) über eine Leitung (15) mit einer Pumpe (17) und einem Ventil (16) und zur Evakuierung über eine Leitung (15, 18) mit einer Pumpe (19) und einem Ventil (20) verbindbar ist.

4. Lithotriptor nach einem der Ansprüche 1, 2 oder 3, dadurch gekennzeichnet, daß die Gaskammer (11) des Ausgleichsbehälters (8) an einen Gasdruckregler (21) angeschlossen ist, mit welchem der Druck des Ankopplungsmediums (2) und somit die Füllhöhe und die Wölbung der Membran (3) einstellbar ist.

## Claims

1. Lithotripter having a leader section of a shock wave transducer (1) filled with a liquid coupling medium (2), sealed by a membrane (3) and which can be placed against a patient, and having a location device comprising an X-ray emitter (4) directed by the leader section and serving for the concretion or tissue to be destroyed by the shock wave transducer, an X-ray-transparent balloon (7) which can be evacuated and filled with gas being sealingly fixed around the output of the X-ray emitter (4) in the leader section, which balloon (7) can be unfolded in the leader section (2) in the direction of emission of the location device, characterised in that an equalising reservoir (8) divided into two chambers (10, 11) by a flexible partition (9) is provided for filling and evacuating balloon (7) with or by gas, one chamber (10) of the equalising reservoir (8) being connected to the coupling medium (2) of the leader section, whereas the other chamber (11) is connected to the balloon (7) for its optional evacuation or filling with gas.

2. Lithotripter according to claim 1, characterised in that the balloon (7) is connected to the open end of a cone (6) which can be filled with gas and is connected to the output of the X-ray emitter (4).

3. Lithotripter according to claim 1 or 2, characterised in that the gas chamber (11) of the equalising reservoir (8) can be connected to a pump (17) and a valve (16) via a pipe (15) to supply gas to the balloon (7), and can be connected to a pump (19) and a valve (20) via a pipe (15, 18) for evacuation.

4. Lithotripter according to one of claims 1, 2 or 3, characterised in that the gas chamber (11) of the equalising reservoir (8) is connected to a gas pressure controller (21), by means of which the pressure of the coupling medium (2) and hence the filling height and the curvature of the membrane (3) can be adjusted.

## Revendications

1. Lithotripteur comprenant un tronçon d'aller d'un transducteur d'ondes de choc (1), qui peut venir s'appliquer contre un patient, qui est fermé par une membrane (3) et qui est rempli d'un milieu liquide d'accouplement (2), ainsi qu'un mécanisme de repérage servant pour la concrétion ou le tissu à détruire à l'intervention du transducteur d'ondes de choc, constitué par un émetteur de rayons X (4) dirigé à travers le tronçon d'aller, dans lequel, autour de la sortie de l'émetteur de rayons X (4) dans le tronçon d'aller, est fixé en étanchéité un ballon (7) transparent aux rayons X, qui peut être rempli avec du gaz et dans lequel on peut faire le vide, qui se déploie dans le tronçon d'aller (2) dans la direction des rayons du mécanisme de repérage, caractérisé en ce que, pour le remplissage du ballon (7) avec du gaz et, respectivement, pour y faire le vide en évacuant le gaz, on prévoit un récipient de compensation (8) subdivisé en deux chambres (10, 11) à l'intervention de n'importe quelle paroi de séparation (9), dont une chambre (10) est raccordée au milieu d'accouplement (2) du tronçon d'aller, tandis que l'autre chambre (11) est reliée au ballon (7) pour pouvoir sélectivement, y faire le vide ou le remplir avec du gaz.

2. Lithotripteur selon la revendication 1, caractérisé en ce que le ballon (7) est raccordé à l'extrémité ouverte d'un tube (6) qui peut être rempli avec le gaz, apte à être raccordé à la sortie de l'émetteur de rayons X (4).

3. Lithotripteur selon la revendication 1 ou 2, caractérisé en ce que la chambre de gaz (11) du récipient de compensation (8), dans le but d'acheminer le gaz au ballon (7), peut être reliée par l'intermédiaire d'un conduit (15) à une pompe (17) et à une soupape (16) et, pour y faire le vide, par l'intermédiaire d'un conduit (15, 18) à une pompe (19) et à une soupape (20).

4. Lithotripteur selon l'une quelconque des revendications 1, 2 ou 3, caractérisé en ce que la chambre de gaz (11) du récipient de compensation (8) est raccordée à un régulateur (21) de la pression du gaz, avec lequel on peut régler la pression du milieu d'accouplement (2) et ainsi, le niveau de remplissage et la courbure de la membrane (3).
